(19)

Europäisches Patentamt

European Patent Office

Office européen des brevets

(11) **EP 2 982 680 B1**

(12) **EUROPEAN PATENT SPECIFICATION**

(45) Date of publication and mention
of the grant of the patent:
**27.02.2019  Bulletin 2019/09**

(51) Int Cl.:
*C07K 7/06* (2006.01)     *A61K 38/08* (2019.01)
*A61K 38/10* (2006.01)     *A61P 7/02* (2006.01)
*C07K 7/08* (2006.01)

(21) Application number: **14778787.3**

(22) Date of filing: **01.04.2014**

(86) International application number:
**PCT/CN2014/000376**

(87) International publication number:
**WO 2014/161370 (09.10.2014 Gazette 2014/41)**

(54) **PROTAMINE PEPTIDOMIMETIC, AND PHARMACEUTICALLY ACCEPTABLE SALTS AND USE THEREOF**

PROTAMINPEPTIDOMIMETIKUM UND PHARMAZEUTISCH AKZEPTABLE SALZE SOWIE VERWENDUNG DAVON

PEPTIDOMIMÉTIQUE DE LA PROTAMINE, SES SELS PHARMACEUTIQUEMENT ACCEPTABLES ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **01.04.2013  CN 201310109830**

(43) Date of publication of application:
**10.02.2016  Bulletin 2016/06**

(73) Proprietors:
• **Academy of Military Medical Sciences**
**Beijing 100850 (CN)**
• **Han, Su**
**Beijing 100850 (CN)**

(72) Inventors:
• **HAN, Su**
**Beijing 100850 (CN)**
• **DOU, Guifang**
**Beijing 100850 (CN)**
• **MENG, Zhiyun**
**Beijing 100850 (CN)**
• **ZHU, Xiaoxia**
**Beijing 100850 (CN)**
• **GAN, Hui**
**Beijing 100850 (CN)**
• **GU, Ruolan**
**Beijing 100850 (CN)**
• **WU, Zhuona**
**Beijing 100850 (CN)**
• **LIU, Taoyun**
**Beijing 100850 (CN)**
• **LI, Jian**
**Beijing 100850 (CN)**
• **ZHENG, Ying**
**Beijing 100850 (CN)**
• **LI, Tong**
**Beijing 100850 (CN)**

(74) Representative: **De Vries & Metman**
**Overschiestraat 180**
**1062 XK Amsterdam (NL)**

(56) References cited:
**WO-A1-92/07871      WO-A1-96/35444
CN-A- 1 263 473      US-A1- 2011 124 584
US-B1- 6 376 248      US-B2- 6 835 808**

• **DELUCIA A III ET AL.: 'Efficacy and toxicity of differently charged polycationic protamine-like peptides for heparin anticoagulation reversal.' JOURNAL OF VASCULAR SURGERY vol. 18, no. 1, 31 July 1993, pages 49 - 60, XP000886143**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Technical Field

The present invention belongs to the field of biomedicine and particularly relates to a linear polypeptide molecule (a protamine-like peptide) which is artificially designed and synthesized and is composed of basic amino acid residues. The polypeptide molecule functions as the protamine to neutralize and antagonize heparin in the blood. Therefore, it is used in clinical practice instead of protamine for eliminating and neutralizing the bleeding tendency caused by the heparin in blood.

## Background of the Invention

Patients with cardiovascular diseases need intravascular injections of heparin (which is an anticoagulant) before undergoing surgeries such as cardiopulmonary bypass or stent intervention to avoid thrombosis during the procedure. However, after the operation, heparin in the blood vessel should be eliminated timely to avoid some adverse bleeding events. Currently, the only anti-heparin drug clinically used is protamine, but the use of protamine has the following disadvantages or drawbacks.

1. Protamine is a protein extracted from the cell nucleus of a mature sperm from the salmon, trout, or herring. It contains a class of protein molecules having a molecular weight of 8000 Daltons or less. The molecular weight of most of the molecules ranges from 4000 to 6000 Daltons. Thus, strictly speaking, protamine is not a pure protein product of a single kind of molecule.

2. Since the finished product of protamine is a group of heterogeneous molecules, the amount sufficient to antagonize the heparin varies depending on the production batches of protamine. In this regard, it is difficult to provide a uniform and consistent quality.

3. Protamine is extracted from mature fish sperms, and the resource of the raw material limits the production of protamine.

4. Since protamine is an exogenous macromolecular protein, human body will be allergic to it. Especially the risk would increase among the patients receiving protamine for the second time, causing a critical medical negligence. Therefore, there are potential hazard and risk in clinical use.

Although the use of protamine involves shortcomings or drawbacks, so far no other alternative to protamine which can antagonize heparin has been found.

## Summary of The Invention

The present invention aims to provide a series of artificially designed and synthesized linear polypeptide molecules consisting of basic amino acid residues, namely protamine-like peptide. The polypeptide molecule functions as protamine to neutralize and antagonize heparin in the blood, and therefore can be used in clinical practice instead of protamine for eliminating or neutralizing the anticoagulation effect caused by heparin in blood.

The protamine-like peptide provided in the present invention is a linear polypeptide consisting of 9 to 20 Arginine residues (R9 to R20), with certain modifications, as defined in the claims. A peptide chain of less than 7 amino acid residues provides no antagonistic effect against heparin while a peptide chain composed of 9 amino acid residues shows antagonistic activity against heparin. The longer the peptide chain of amino acids is, the higher the antagonistic activity against heparin will be. On the other hand, the antagonistic effect of the peptide chain on heparin is positively correlated with the alkalinity of the amino acids forming the peptide chain. That is, among peptide chains with the same number of amino acid residues, a peptide chain purely consisting of Arginine residues has a superior antagonistic effect against heparin to that of one purely consisting of Lysine residues alone. Further, the antagonistic effect against heparin of a peptide chain consisting of Lysine residues alone is superior to that of one consisting of Histidine residues alone.

Said protamine-like peptide is derived from a peptide chain composed of 9 to 20 (particularly preferably 15) Arginine residues with one Arginine residue therein at any position being replaced with one Histidine residue (i.e., a linear long peptide consisting of 14 Arginine residues and 1 Histidine residue, which is referred to as R15-H). It is convenient to perform an isotope (such as iodine) labeling in such a protamine-like peptide which functions as a pharmaceutical molecule.

In addition, all the salt forms (pharmaceutical salts) derived from the series of the protamine-like peptides described above are involved in the present invention. The salt form includes the inorganic salt such as sulfate, acetate, phosphate, hydrochlorate, carbonate or the like; and the organic salt such as maleate, citrate, tartrate, sulfonate, salicylate, malate or the like. The antagonistic effect on heparin will not be affected when the series of the protamine-like peptides are in an inorganic salt form or in an organic salt form.

Another objective of the present invention is to provide the use of said series of protamine-like peptides and salt forms thereof in the preparation of a heparin-antagonizing drug. This drug is used in clinical practice to eliminate and neutralize the bleeding tendency caused by heparin in blood.

Compared to protamine, the artificially designed and synthesized linear polypeptide molecule composed of amino acid residues in the present invention (protamine-like peptide) has the following advantages:

1. The protamine-like peptide and the salt thereof in the present invention have well defined efficacy and show effect on neutralizing and antagonizing heparin in blood.

2. The protamine-like peptide of the present invention is an artificially designed polypeptide molecule which is composed of basic amino acid residues and has well defined amino acid constitution and molecular sequence. Thus, it is easy to be artificially synthesized.

3. The protamine-like peptide of the present invention can be artificially synthesized in a short time in a large scale with a high purity, which not only gets rid of the problem related to natural fish resource but also meets clinical needs for large quantity.

4. The protamine-like peptide of the present invention has a smaller molecular weight than protamine and the molecular weight is about 1500 to 3000 Daltons. After purification by high performance liquid chromatography (HPLC), the purity of the peptide becomes higher than 99.0%, which meets the international purity standard for pharmaceutical peptide preparation. This reduces sensitization rate in clinical use, and therefore it has a higher safety than the natural protamine.

5. Since the protamine-like peptide of the present invention consists of a single kind of molecule, the dose to be administrated will be more rigorous and precise, bringing more accurate effect.

Studies have shown that the above-mentioned series of protamine-like peptides and any salt forms thereof have an effect on neutralizing and antagonizing heparin in blood and therefore may replace protamine. A heparin-antagonizing drug may be prepared by using the series of protamine-like peptides or any salt forms thereof as the active ingredient(s) so as to eliminate and neutralize the bleeding tendency caused by the circulating heparin in clinical practice.

Based on the advantages mentioned above, the protamine-like peptide of the present invention can be used clinically instead of protamine to eliminate and neutralize heparin-induced bleeding tendency and has a unique role in clinical practice.

Hereinafter, the present invention will be further described in details using the specific Examples.

## Brief Description of the Drawings

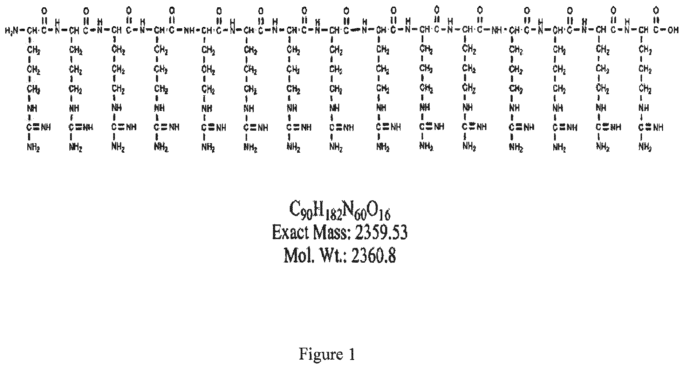

Figure 1 is a drawing showing the structure of the linear protamine-like peptide R15 purely consisting of Arginine residues.

## Detailed Description of the Invention

The genes in an organism are stored in the polynucleotide chains and encode proteins which perform biological functions. Many different proteins exist in an organism and perform a variety of biological functions to maintain the life activities. Despite of various kinds, the proteins are basically composed of 20 kinds of naturally occurring amino acids. Differences in the composition and arrangement order of the amino acid residues result in vastly different proteins. Generally speaking, a molecule containing 50 or more amino acid residues is referred to as a protein, and a peptide chain of more than 10 amino acid residues is known as a polypeptide. In addition, a peptide chain of less than 10 amino acid residues is referred to as an oligopeptide. The smallest functional peptide chain found up to now consists of only two amino acid residues.

As a result of the completed Human Genome Project (HGP) and the ongoing Human Proteome Project (HPP), more and more functional fragments of protein have been and are being identified and used as drugs in the biomedical field. Polypeptide drugs which have already been utilized in clinical practice include "oxytocin", "thymosin α1", "thymopentin" or the like. Also, there are polypeptide drugs prepared by engineering on the basis of natural peptide chain, such as "octreotide" used to treat gastrointestinal bleeding and acromegaly, and "hirudin" with anti-clotting effect. The functional fragments in the proteins can often be screened to provide polypeptide fragments of dozens of amino acid residues or of only two amino acid residues which are actually artificially synthesized functional polypeptide fragments. This lays the ground for their applications.

In 1963, Professor Robert Bruce Merrifield from Rockefeller University developed a solid phase peptide synthesis method which is a revolutionary organic solid-phase synthesis method. This method can synthesize polypeptides in a quick, convenient and efficient way. On the basis of this, mechanically automatic synthesis of polypeptides was enabled, and the prototype of the automated polypeptide synthesizer was first introduced in the late 1960s. With the technique progresses and the demands for polypeptide studies, the polypeptide synthesizer has been divided into microgram, milligram, gram and kilogram classes in terms of the synthesis throughput; research, pilot, pilot scale experiment, normal production and GMP production types in terms of function; fully automatic, semi-automatic and manual types in terms of the level of automation; single-channel and multi-channel synthesizers in terms of channels. Professor R. Merrifield has been awarded the 1984 Nobel Prize in Chemistry for this.

Polypeptide synthesis has now become a commercial technical service, and services such as polypeptide synthesis and modification may be provided in accordance with customer's demands.

With the modern technologies, the present invention provides a series of artificially designed and synthesized protamine-like peptides which are made to have an anti-heparin activity in a same or similar level to that of protamine.

Protamine is a basic protein extracted and isolated from the fish spermary. It is widely used in clinic and is the most commonly agent used to overcome the toxic effect induced by heparin.

As described in the literatures, its amino acid sequence is PRRRRSSSRPVRRRRRPRVSRRRRRRGGRRRR (P represents Proline, R represents Arginine, S represents Serine, V represents Valine, and G represents Glycine), and the molecular weight is about 3800 Da. As is known, one amino acid sequence of protamine has been identified. Nevertheless, protamine is an extract from the natural fish spermary and is actually a gathering of heterogeneous molecules. The composition is complex and the structure is unsure. In fact, it is hard to reproduce the protamine artificially. In the present invention, the term "XX-like" refers to an alternative product having the same or similar anti-heparin functions as protamine which is artificially designed on the basis of the well known sequence information of the protamine.

In order to clearly represent the present invention, the details will be described in the following paragraphs. The methods used in the Examples or Tests are conventional ones unless otherwise specified. Reference may be made to Research and Development of Polypeptide Drugs, edited by Li Baoqiu, People's Medical Publishing House, Co., Ltd., 2011-07, and Bioassay of Protamine Sulfate from China Standard Operation Procedure for Drug Test, 2010 edition, China Pharmaceutical Press.

The means to obtain various biological materials as mentioned in Examples or Tests are listed here only to provide a possible way where materials are obtained through experiments and for the purpose of particular disclosure, and therefore should not be deemed as the restriction on the sources of biological materials in the present invention. In fact, the biological material used here is obtainable from broad origins, and any biological materials available without acting against any law and ethics can be alternatively used in accordance with instructions listed in Examples.

The examples are implemented on the basis of the technical solution of the present invention and provide detailed embodiments and specific operating procedures. Examples will help to understand the present invention and provides more about the design, the features, the efficacy and particular applications of the polypeptides of the present invention. However, the protection scope of the present invention is not limited to the following examples.

Reagents or materials used in Examples and Tests of the present invention have the same meanings unless otherwise specified.

Example 1: Design of protamine-like peptide (for reference, not part of the claimed invention)

Design ideas and methods:

Heparin was a natural anti-clotting substance extracted from bovine lungs or porcine small intestinal mucosa. In particular, it was a sulfated glycosaminoglycan with an average molecular weight of 15 KD and was strongly acidic with a large quantity of negative charges. Based on the chemical properties of heparin, the present invention employed a polycationic protamine-like peptide consisting of basic amino acid residue to neutralize or antagonize the polyanionic heparin.

In terms of alkalinity, three basic amino acids were arranged in the following order, i.e., Arginine (Arg, R) > Lysine (Lys, K) > Histidine (His, H). Through theoretical design and effective experimental tests, the present invention finally identified a series of artificially designed and synthesized protamine-like peptides. They were linear polypeptides composed of three kinds of basic amino acids with a total of 9 to 20 amino acid residues, wherein the basic amino acids were Aginine (Arg, R), Lsine (Lys, K) and Hstidine (His, H). Here, these three kinds of basic amino acids can be located at random positions and can be combined at any ratio. Specifically, the followings were provided.

Group A: This series of protamine-like peptides were linear polypeptides composed of Arginine residues (Arg, R) alone with a number of 9 to 20 (referred to as R9 to R20). A peptide chain with less than 7 Arginine residues had no antagonistic effect on heparin whereas a peptide chain of 9 Arginine residues began to show antagonistic activity against heparin. The longer the peptide chain of Arginine residues was, the higher the antagonistic activity against heparin would be.

Group B: This series of protamine-like peptides were linear polypeptides composed of Lysine residues (Lys, K) alone with a number of 9 to 20 (referred to as K9 to K20). A peptide chain with less than 7 Lysine residues had no antagonistic effect on heparin whereas a peptide chain of 9 Lysine residues began to show antagonistic activity against heparin. Within the range of K9 to K 20, the longer the peptide chain of Lysine residues was, the higher the antagonistic activity against heparin would be.

Group C: This series of protamine-like peptides were linear polypeptides composed of Histidine residues (His, H) alone with a number of 9 to 20 (referred to as H9 to H20). A peptide chain with less than 7 Histidine residues had no antagonistic effect on heparin whereas a peptide chain of 9 Histidine residues began to show antagonistic activity against heparin. Within the range of H9 to H20, the longer the peptide chain of Histidine residues was, the higher the antagonistic activity against heparin would be.

Tests demonstrated that the peptide chains composed of a single type of basic amino acid (Arginine, Lysine or Histidine) (Group A to Group C) exhibited antagonism against heparin and the anti-heparin effect provided by these peptide chains was positively correlated with the alkalinity of the amino acid forming the peptide chain (see Test 2). That is, among peptide chains with the same number of amino acid residues, a peptide chain consisting of Arginine residues alone (Group A) showed a superior antagonistic effect against heparin to that of one consisting of Lysine residues alone (Group B). Furhter, the antagonistic effect against heparin of a peptide chain consisting of Lysine residues alone (Group B) was superior to that of one consisting of Histidine residues alone (Group C).

Group D: This group concerned the polypeptide of mixed types of amino acids. As peptide chains from Group A to Group C were all active, the peptides from only one of these groups were picked as the basic peptide chains where the amino acid residue(s) at one or more random positions were replaced with another one or other two kinds of amino acid residues so as to provide a target linear polypeptide. Calculated based on mathematical permutations and combinations, the number of polypeptides formed in group D was tremendous. In Table 1, an example was provided where 9 Arginine residues formed the basic peptide chain and two amino acid residues were changed. In this way, the compositions in the group involving polypeptides of mixed kinds of amino acids were intuitively provided to help understand the idea. Obviously, the polypeptides had not been presented in Table 1 in an exhaustive manner.

Table 1: Mixed amino acid residues with two out of nine Arginine residues being changed

| Indication | Polypeptide sequence |
|---|---|
| R9-2H | RRRHHRRRR |
| | RHHRRRRRR |
| | RHRRRRRHR |
| | RRHRRRHRR |
| R9-2K | KRRRRRRRK |
| | RKRRRRKRR |
| | RRRKRKRRR |
| R9-1H1K | KRRRHRRRR |
| | RRRHRKRRR |
| | RRRRRKHRR |
| | RRHRRRKRR |

On the other hand, in the group involving polypeptides of mixed amino acid residues, the polypeptides also had antagonism against heparin (see Test 4). With respect to the polypeptides with different numbers of amino acid residues, the anti-heparin activity increased as the number increased (see Test 1). Further, it was predicted that among polypeptides with the same number of amino acid residues, the alkalinity of the amino acid residues composing the polypeptide (alkalinity: R> K> H) was positively correlated with the anti-heparin activity. For example, in Table 1, the activity was as follows, i.e., R9-2K> R9-1H1K> R9-2H.

Group E: In peptide chains R9 to R20 purely consisting of Arginine residues, one Arginine residue at any one

5

position was replaced with one Histidine residue. This group was designed to facilitate the tracing of the drug molecule through isotope (iodine) labeling.

In order to conveniently track the hemodynamic metabolism of drug molecules, in a peptide chain mainly composed of Arginine residues (for example, R15), the Arginine residue(s) at any position(s) was/were substituted by one Histidine residue which might be labeled with an iodine isotope ($H^{I-125}$ or $H^{I-131}$) so as to produce a polypeptide with the sequence as set forth in Table 2.

Table 2: Mixed amino acid residues with one out of fifteen Arginine residues being changed to Histidine residue

| Indication | Polypeptide sequence |
|---|---|
| R15-H | RRRRRHRRRRRRRRR (H in the middle, R15-8) |
| | RRRRRRRRRRHRRRR (H off-center, R15-5) |
| | RRRRRRRRRRRHRRR (H off-center, R15-4) |
| | RRRRRRRRRRRRHRR (H off-center, R15-3) |
| | RRRRRRRRRRRRRHR (H off-center, R15-2) |
| Note: In Table 2, H was not arranged at the terminal of the peptide to avoid the loss of the isotope labeled H during the carboxypeptidase-catalyzed polypeptide degradation which would affect the molecule tracking. | |

In a protamine-like peptide consisting of Arginine residues and one Histidine residue, the position where the Histidine residue was located in the peptide chain did not evidently interfere with the antagonistic effect of the peptide against heparin (see Test 3 for R15). Therefore, in a peptide chain of basic amino acid residues with residues mainly being Arginine residues, the Histidine residue can be located at any position, which would not significantly affect anti-heparin activity of the protamine-like peptide.

Group F: This group involved preferred protamine-like peptides.

According to the experimental test results, among numerous peptide chains being tested, preferable protamine-like peptides were selected in accordance with the following approaches.

1. Among effective polypeptide chains, a polypeptide with a relatively short chain was to be selected, which helped to reduce antigenicity of exogenous peptide molecules and facilitate rapid degradation of excess peptide molecules remaining after heparin neutralization.

2. Among peptide chains of three types of basic amino acids, a peptide chain consisting of the most basic amino acid residues was to be picked. A peptide chain purely consisting of Arginine residues was selected to neutralize or antagonize the strongly acidic heparin.

The larger the molecular weight of a peptide drug was, the more likely the antibody generation and adverse reactions would occur in a body. If an overall consideration was taken on factors such as the antagonistic effect against heparin and input-output ratio, the peptide chain should be as short as possible for a clinical medicine. According to this idea, the recommended preferred protamine-like peptides included R16, R15, R14, R13, R12, R11, R10 or the like and the suitable pharmaceutical salts thereof.

The present invention specifically proposed a preferred protamine-like peptide which was polypeptide chain R15 purely consisting of Arginine residues. Its structure was shown in Figure 1.

Tests had confirmed that (see Test 6) the protamine-like peptide R15 had a comparable anti-heparin activity to protamine and can be used as an alternative to protamine (extraction product) to clinically eliminate and neutralize the clotting caused by heparin in blood.

Group G: Pharmaceutical salt of protamine-like peptide

In the design of the series of protamine-like peptides mentioned above, the compounds in the salt form thereof had also been taken into account. Said salt forms included the inorganic salt such as sulfate, acetate, phosphate, hydrochlorate, carbonate or the like; the organic salt such as maleate, citrate, tartrate, sulfonate, salicylate, malate or the like. It was proved that neither the inorganic salt nor the organic salt of these protamine-like peptides had its anti-heparin activity affected (See Test 5).

Example 2: Synthesis of protamine-like peptide

In 1963, R.B. Merrifield, an American scientist, invented a solid phase synthesis method in which the carboxyl terminus (C terminal) of the target peptide was fixed on an insoluble resin, and the amino terminus (N terminal) of the amino acid bound to the resin underwent a condensation reaction with the carboxyl terminus to be joined so as to extend the peptide chain. The polypeptide synthesis started from the carboxyl terminus (C terminal) of the polypeptide and extended to the amino terminus (N terminal) of the peptide segment by condensing amino acids one by one. During conjugation reaction of the carboxyl group in the amino acid, its amino group and the side chain group(s) should be protected to avoid reactions, and currently the most commonly used methods were t-butyloxycarbonyl (Boc) protection and fluorenylmethyloxycarbonyl (Fmoc) protection. Therefore, whenever an amino acid was connected, the amino group of the amino acid bound to the solid phase support was subjected to a deprotection process followed by a condensation reaction with excess activated carboxyl group from the amino acid to be connected, extending the peptide chain. This step was repeated again and again, namely the condensation -> rinsing -> deprotection -> neutralization and rinsing -> next round of condensation (to add another amino acid), until a desired length was achieved for the synthetic peptide. Upon the completion of polypeptide synthesis, the synthesized polypeptide product was cleaved off from the solid phase resin and then purified by high performance liquid chromatography to yield the final synthetic product. Based on such a principle, the polypeptide synthesis can be either manually operated or automatically synthesized with a polypeptide synthesizer by inputting the sequence to be synthesized and the corresponding programs.

The synthesis of the series of protamine-like peptides in the present invention used the solid phase synthesis method with amino acids being chemically protected by Fmoc. Using resin WANG (purchased from GL Biochem (Shanghai) Ltd.), in an automated polypeptide synthesizer (model ABI433A), the synthesized linear polypeptide extended by condensation of one amino acid at one time from the carboxyl terminus (C) of the polypeptide to the amino terminus (N). After the synthesis was completed, with TFA (trifluoroacetic acid) method (for details, refer to Chillemi F. Merrifield R B. Use of N-im-dinitrophenylhistidine in the solid-phase synthesis of the tricosapeptides124-146 of human hemoglobin, Biochemistry, 1969, (8): 4344), the target polypeptide was cleaved from the resin and then purified by high performance liquid chromatography (HPLC) where the specific parameters were as follows. A $C_{18}$ column (10 $\mu$m, 100 Å, 50 $\times$ 250 mm) from Daiso Co. Ltd., Japan was used, the mobile phase A was an aqueous solution containing 0.05% of trifluoroacetic acid and 2% of acetonitrile, and the mobile phase B was 90% acetonitrile / water. The flow rate was set as 25 ml per minute and the wavelength in ultraviolet (UV) detection was 220 nm. The eluting peak solution was collected and then lyophilized to yield a white flocculent solid polypeptide product.

When the target polypeptide was obtained by the solid phase synthesis method, the polypeptide molecule was purified by high performance liquid chromatography to yield a polypeptide product with purity higher than 99.0%.

The Series of protamine-like peptides designed in Example 1 could all be synthesized as described above. The present invention focused on the design for the amino acid sequence of the polypeptide molecule and also the application thereof, and specific procedures in polypeptide synthesis (which were not described in detail) were carried out by conventional means according to design.

Test 1. Test on antagonism of series of protamine-like peptides with a different number of amino acid residues against heparin anticoagulation in plasma *in vitro* (for reference, not part of the claimed invention)

Purpose:

This test aimed to test antagonism of series of synthetic protamine-like peptides against heparin anticoagulation in plasma *in vitro*.

Mechanism:

This test mainly aimed to test anti-heparin activity of six protamine-like peptides with different numbers of amino acid residues. The principle where the heparin antagonist functioned was that the positive charges carried by the protamine neutralized the negative charges in heparin bound to antithrombin III (AT-III) so as to release the antithrombin III (AT-III). The released antithrombin III activated coagulation factors IIa, IXa, Xa, XIa, and/or XIIa and promoted the generation of thromboplastin, turning the prothrombin to thrombin which expedited the conversion of fibrinogen to fibrin.

Materials:

Six kinds of protamine-like peptides, R3, R5, R7, R9, R15 and R20, were synthesized by the solid phase synthesis method, wherein R represented Arginine (Arg). The purity was higher than 99.0% and the lot number was set as 20130321. The peptides had good water solubility. After dissolved in water, the peptides were sub-packaged and lyophilized to yield white floccules.

Heparin sodium: Purchased from Sinopharm Chemical Reagent Co., Ltd. with a lot number of F20110919, the specific activity being 150 U/mg.

Protamine sulfate injection: Purchased from Yuekang Hyatt Beijing Pharmaceutical Group Co., Ltd., State Medical Permitment No. being H11020246, 09121002, expiration date being September 2015; average molecular weight being about 3800 Da.

Whole blood for test: Fresh animal whole blood taken from New Zealand rabbits (purchased from Military Academy of Medical Sciences Laboratory Animal Center).

Methods:

Preparation of solution of heparin reference standard

Reference standard of heparin was exactly weighed and then dissolved into several solutions with different concentrations by adding 0.9% sodium chloride solution according to the marked potency. The increment of heparin units per mL between two solutions having adjacent concentrations should be similar with no more than five units. Generally, solutions containing 85, 90, 95, 100, 105, 110, 115, 120, 125 units of heparin per mL could be prepared.

Preparation of solution of test samples

The test sample was the protamine-like peptide. If the test sample was in a powder form, it was accurately weighed on the basis of dry sample. Then, a solution of 0.9% sodium chloride was added thereto to provide a solution containing 1 mg of test sample per mL.

Assay

Eight small dry clean teat tubes with the same diameter (0.8 × 3.8 cm) were picked. Tube 1 and Tube 8 were used as the blank control into which 0.2 mL of 0.9% sodium chloride solution was added. Tube 2 to Tube 7 were used as the ones with test samples into each of which 0.1 ml of test solution was added together with 0.1 ml of heparin standard dilution having one concentration mentioned above. The mixture was well mixed immediately. An appropriate amount (0.8 ml) of whole blood freshly drawn from rabbits was added into these eight test tubes described above and then well mixed immediately to avoid generation of bubbles. Timing began at that time and the test tubes were all placed in a thermostatic water bath at 37 ± 0.5 °C. The interval between blood sampling and tube incubation in the thermostatic water bath should not be longer than two minutes. If the plasma was used, then 0.7 mL of plasma was added to each of the test tubes described above and then the test tubes were placed into a thermostatic water bath at 37 ± 0.5 °C to warm up for 5 to 10 minutes. Then, into each test tube, 0.1 mL of 1% calcium chloride solution was added and immediately mixed to avoid generation of bubbles. At that time, timing began. The test tubes were observed and the time until coagulation was recorded.

Results

The clotting time in one control tube should not be more than 1.35 times longer than that in the other control tube. Among test tubes where the clotting time was no longer than 150% of the average coagulating time in two control tubes, the tube with the highest concentration of heparin was used as the endpoint tube.

The test was repeated for five times. The concentration of heparin in the endpoint tube was measured in all five tests, and the deviation was not allowed to be higher than 10 units. The average from five tests was deemed as the number of units of the heparin neutralized by 1 mg of the test sample (dry product) (the amount of neutralized heparin).

Data processing for Rabbit whole blood:

The anticoagulant time of heparin, i.e. the clotting time from the addition of the series of protamine-like peptides, were recorded where the clotting time in tubes with plasma but not heparin was considered as the control clotting time. The average value in each coagulation test was calculated, and ratio of the concentration in nmol of the added potamine-like peptide to 1 IU of neutralized heparin was regarded as the anti-heparin index (I) of the protamine-like peptide.

$$\text{Anti-heparin index} = \text{concentration of polypeptide} / 1 \text{ unit of heparin (nmol / U)}.$$

Assay results of six protamine-like peptides R3, R5, R7, R9, R15 and R20 were shown in Table 3.

Table 3. Number of units of heparin neutralized by protamine-like peptide in rabbit whole blood added with heparin and Anti-heparin index

| | Sequence | Molecular weight | Amount of neutralized heparin (U/mg) | | | | | | Clotting time (min.) | I |
| --- | --- | --- | --- | --- | --- | --- | --- | --- | --- | --- |
| | | | 1 | 2 | 3 | 4 | 5 | Mean $\pm$ SD | | |
| Commercially available protamine | | 3800 | 105 | 105 | 100 | 100 | 100 | 102$\pm$2.45 | 13.14$\pm$1.34 | 2.58 |
| R3 | $(R)_3$ | 486.58 | - | - | - | - | - | - | - | - |
| R5 | $(R)_5$ | 798.96 | - | - | - | - | - | - | - | - |
| R7 | $(R)_7$ | 1111.34 | - | - | - | - | - | - | - | - |
| R9 | $(R)_9$ | 1423.72 | 70 | 70 | 70 | 75 | 70 | 71$\pm$2.24 | 14.44$\pm$1.17 | 12.67 |
| R15 | $(R)_{15}$ | 2360.86 | 115 | 110 | 110 | 110 | 115 | 112$\pm$2.74 | 12.69$\pm$0.44 | 6.27 |
| R20 | $(R)_{20}$ | 3141.81 | 108 | 106 | 110 | 100 | 109 | 106.6$\pm$3.97 | 12.18$\pm$0.45 | 4.00 |
| Whole blood (blank control) | - | - | - | - | - | - | - | - | 10.62$\pm$0.49 | - |
| I = Anti-heparin index; R = Arginine; - = Not applicable or incalculable | | | | | | | | | | |

EP 2 982 680 B1

Conclusion:

The test results indicated that R9, R15 and R20 exhibited anti-heparin effects. For the peptide chain purely consisting of Arginine (R) residues, as the number of Arginine residues increased (i.e. from 9 Arginine residues (R9) to 20 Arginine residues (R20)), the molecular weight increased and the anti-heparin index declined, indicating a better heparin-antagonizing effect.

The inventors further tested polypeptides with more than 20 Arginine residues, and the result showed that R21 to R25 and polypeptides with more than 25 Arginine residues had a significant heparin-antagonizing effect. Therefore, with regard to anti-heparin capacity, there was no upper limit for the number of amino acid residues in theory. However, if the synthesis and industrial application were to be considered, amino acid residues should not be too many.

On the other hand, the inventors conducted the same experiment for R8 with animal blood to test whether R7 and R9 outlined in Table 3 were the watersheds of heparin antagonism. The results (see Table 4) showed that the clotting time provided by R8 in whole blood was much longer than that in the blank control with whole blood, indicating a relatively weak anti-heparin activity. However, the activity was less than 70 U/mg.

Overall, the present invention determined R9 to R20 as the proper peptides.

Table 4. Number of units of heparin neutralized by protamine-like peptide R8 in rabbit whole blood added with heparin and Anti-heparin index

| | | | Amount of neutralized heparin (U/mg) | | | | | | Clotting time (min.) | I |
|---|---|---|---|---|---|---|---|---|---|---|
| | Sequence | Molecular weight | 1 | 2 | 3 | 4 | 5 | Mean $\pm$ SD | | |
| Commercially available protamine | | 3800 | 105 | 105 | 100 | 100 | 100 | 102$\pm$2. 45 | 13.14$\pm$1.34 | 2.58 |
| R8 | (R)$_8$ | 1267.52 | <70 | <70 | <70 | <70 | <70 | - | 16.38$\pm$3.58 | - |
| Whole blood (blank control) | - | - | - | - | - | - | - | - | 10.62:1:0.49 | - |

The same test was carried out with the same method for K3 to K20 involving Lysine and H3 to H20 involving Histidine, resulting in the same conclusion. For conciseness, experimental procedures and corresponding data were not provided here.

This test had demonstrated that

1. The peptide chain with less than seven amino acid residues had no evident heparin-antagonizing effect.

2. The peptide chain composed of eight amino acid residues began to exhibit an anti-heparin activity, and the peptide chain with nine or more amino acid residues was more effective. In addition, as the peptide chain became longer, the anti-heparin activity increased. It had been determined that the protamine-like peptides within the range of R9 to R20, K9 to K20 or H9 to H20 were suitable.

3. The biological activity of the protamine-like peptide varied depending on the length of the peptide chain, and the activity was close to or slightly weaker than that of commercially available protamine.

Test 2. Test on antagonism of peptides purely consisting of each of three basic amino acid residues against heparin anticoagulation in plasma *in vitro* (for reference, not part of the claimed invention)

Purpose:

This test aimed to test the effect of three kinds of basic amino acids, i.e. Arginine (Arg, R), Lysine (Lys, K) and Histidine (His, H), on the heparin antagonism. Especially, the synthetic R15 (a peptide chain containing 15 Arginine residues), K15 (a peptide chain containing 15 Lysine residues) and H15 (a peptide chain containing 15 Histidine residues) were compared for the heparin-antagonizing effect.

Materials:

Three kinds of protamine-like peptides, R15, K15 and H15, were synthesized by the solid phase synthesis method. They had a purity higher than 99.0% and good water solubility. After dissolved in water, the peptides were sub-packaged and lyophilized to yield white floccules.

Heparin sodium: Purchased from Sinopharm Chemical Reagent Co., Ltd. with a lot number of F20110919, the specific activity being 150 U / mg.

Protamine sulfate injection: Purchased from Yuekang Hyatt Beijing Pharmaceutical Group Co., Ltd., State Medical Permitment No. being H11020246, 09121002, expiration date being September 2015.

Animals: Rabbit purchased from Military Academy of Medical Sciences Laboratory Animal Center.

The test method and data processing were the same as in Test 1. The test results were shown in Table 5.

Table 5. Comparison on Number of units of heparin neutralized by protamine-like peptides R15, K15 and H15 in rabbit whole blood added with heparin and Anti-heparin index

| | Sequence | Molecular weight | Amount of neutralized heparin (U/mg) | | | | | | Clotting time (min.) | I |
|---|---|---|---|---|---|---|---|---|---|---|
| | | | 1 | 2 | 3 | 4 | 5 | Mean ± SD | | |
| Commercially available protamine | | 3800 | 105 | 105 | 100 | 100 | 100 | 102±2.45 | 13.14±1.34 | 2.58 |
| R15 | (R)$_{15}$ | 2360.86 | 115 | 110 | 110 | 110 | 115 | 112±2.74 | 12.69±0.44 | 6.27 |
| K15 | (K)$_{15}$ | 1940.56 | 80 | 82 | 83 | 85 | 83 | 82±1.86 | 18.35±0.33 | - |
| H15 | (H)$_{15}$ | 2075.13 | <70 | <70 | <70 | <70 | <70 | <70 | >30 | - |
| Whole blood blank | - | - | - | - | - | - | - | - | 10.62±0.49 | - |
| I = Anti-heparin index; R = Arginine; H = Histidine; K = Lysine; - = Not applicable or incalculable According to the pKa value of the amino acid, these three basic amino acids were arranged as follows in terms of alkalinity: Arginine (Arg, R) (pKa=12.5) > Lysine (Lys, K) (pKa=10.5) > Histidine (His, H) (pKa=6.0). | | | | | | | | | | |

Based on the isoelectric point (pI) of the amino acid, they were arranged as follows: Arginine (Arg, R) (pI=10.76) > Lysine (Lys, K) (pI=9.74) > Histidine (His, H) (pI=7.59).

This test demonstrated that all the peptides composed of 15 basic amino acid residues of a single kind (Arginine, Lysine or Histidine) showed antagonism or neutralization against heparin. The anti-heparin effect of the peptide chain was positively correlated with the alkalinity of the amino acids composing the peptide chain. In particular, the antagonistic effect against heparin of a peptide consisting of Arginine residues alone was superior to that of the one consisting of Lysine residues alone, and the antagonistic effect against heparin of a peptide consisting of Lysine residues alone was superior to that of one consisting of Histidine residues alone.

Test 3. Test on effect of Histidine residue at different positions of the protamine-like peptide on heparin antagonism

Purpose:

In order to develop a clinical medicine for human body, the isotopically labeled Histidine was set up in the anti-heparin molecule to conveniently study the metabolism of the medicine. Here, the effect of Histidine residue at different positions of the protamine-like peptide on heparin antagonism was tested.

Materials:

Three kinds of protamine-like peptides with purity higher than 99.0% and a good water solubility were synthesized through the solid phase synthesis method, wherein R15-2, R15-5 and R15-8 were randomly selected from Table 2. After dissolved in water, the peptides were sub-packaged and lyophilized to yield white floccules.

Other materials, test method and data processing were the same as in Test 1.

The test results were shown in Table 6.

In a polypeptide chain consisting of the basic amino acid residues, the capacity to carry positive charges was better in Arginine (Arg, R) than Lysine (Lys or K) and Histidine (His or H). In this test, one Arginine residue in a strongly basic amino acid peptide chain consisting of Arginine residues alone was changed to a Lysine residue (Lys, H) so that isotope can be easily labeled in this polypeptide molecule. In this way, the drug metabolism and the distribution of the drug in tissues can be tracked. The test indicated that in protamine-like peptide consisting of Arginine residues and one Histidine residue, the position in the peptide chain where the Histidine residue was located (either at position 2, 5 or 8) did not significantly interference with heparin antagonism. Therefore, the Histidine residue can be located at any position, which would not produce an evident effect on the heparin-antagonizing effect of the protamine-like peptide.

Table 6. Comparison on Number of units of heparin neutralized by protamine-like peptides R15-H in rabbit whole blood added with heparin and Anti-heparin index

| Test sample | Sequence | Molecular weight | Amount of neutralized heparin (U/mg) | | | | | | Clotting time (min.) | I |
|---|---|---|---|---|---|---|---|---|---|---|
| | | | 1 | 2 | 3 | 4 | 5 | Mean $\pm$ SD | | |
| Commercially available protamine | | 3800 | 105 | 105 | 100 | 100 | 100 | 102$\pm$2.45 | 13.14$\pm$1.34 | 2.58 |
| R15 | $(R)_{15}$ | 2360.86 | 115 | 110 | 110 | 110 | 115 | 112$\pm$2.74 | 12.69$\pm$0.44 | 6.27 |
| R15-2 | $(R)_1 (H)_1 (R)_{13}$ | 2341.81 | 110 | 113 | 110 | 110 | 110 | 110$\pm$1.342 | 13.35$\pm$0.56 | 4.66 |
| R15-5 | $(R)_4 (H)_1 (R)_{10}$ | 2341.81 | 110 | 113 | 105 | 110 | 105 | 118.6$\pm$1.34 | 14.20$\pm$035 | 3.86 |
| R15-8 | $(R)_7 (H)_1 (R)_7$ | 2341.81 | 110 | 105 | 105 | 112 | 110 | 108.4$\pm$1.32 | 14.35$\pm$0.38 | 3.93 |
| Whole blood (blank control) | - | - | - | - | - | - | - | - | 10.62$\pm$0.49 | 3.94 |
| I = Anti-heparin index; R = Arginine; H = Histidine; - = Not applicable or incalculable | | | | | | | | | | |

It can be seen that in a peptide with mixed kinds of basic amino acids, the residue different from the major ones may be located at any position, which would generally produce no evident effect on the anti-heparin activity of the protamine-like peptide.

Test 4. Test on antagonism of long peptides with three mixed kinds of basic amino acids against heparin anticoagulation in plasma *in vitro*

For the test method, please refer to that described in Test 1. Long liner peptides synthesized with three kinds of basic amino acids through the solid phase synthesis method were used as the test materials, as shown in Table 6. The number of units of heparin neutralized by protamine-like peptides consisting of mixed amino acids in rabbit whole blood added with heparin and the anti-heparin index were shown in Table 7.

As can be seen, among peptides with mixed basic amino acids, two kinds of amino acids other than Arginine may be incorporated into a long peptide consisting of 10 to 20 Arginine residues, and the resulting protamine-like peptide also had anti-heparin activity.

Table 7. Comparison on Number of units of heparin neutralized by protamine-like peptides consisting of mixed amino acids in rabbit whole blood added with heparin and Anti-heparin index

| No. | Sequence | Molecular weight | Amount of neutralized heparin (U/mg) | | | | | | I | Clotting time (min) |
|---|---|---|---|---|---|---|---|---|---|---|
| | | | 1 | 2 | 3 | 4 | 5 | Mean $\pm$ SD | | |
| Commercially available protamine | - | About 3800 on average | 105 | 105 | 100 | 100 | 100 | 102$\pm$2.45 | 2.58 | 13.14$\pm$1.34 |
| R6KH | $(R)_3K_1H_1(R)_3$ | 1220.46 | < 60 | < 60 | < 60 | < 60 | < 60 | < 60 | (-) | - |
| R10KH | $(R)_5K_1H_1(R)_5$ | 1845.21 | 90 | 90 | 90 | 95 | 90 | 91$\pm$2.24 | 5.93 | 8.47$\pm$0.32 |
| R14KH | $(R)_7K_1H_1R_7$ | 2469.96 | 120 | 115 | 115 | 115 | 115 | 116$\pm$2.24 | 3.49 | 8.72$\pm$0.51 |
| R18KH | $(R)_9K_{41}H_1(R)_9$ | 3094.71 | 110 | 110 | 110 | 110 | 115 | 111$\pm$2.24 | 2.91 | 9.06$\pm$0.56 |
| R20KH | $(R)_{10}K_1H_1(R)_{10}$ | 3407.08 | 110 | 115 | 120 | 115 | 115 | 115$\pm$3.53 | 2.71 | 9.35$\pm$1.06 |
| Whole blood (blank control) | - | | - | - | - | - | - | - | - | 10.62$\pm$0.49 |
| I = Anti-heparin index; R = Arginine; H = Histidine; K = Lysine; - = Not applicable or incalculable | | | | | | | | | | |

Test 5. Test on antagonism of salts of series of protamine-like peptides against heparin anticoagulation in plasma *in vitro* (for reference, not part of the claimed invention)

Purpose:

The protamine-like peptides of the present invention were polypeptides composed of basic amino acid residues and had the nature of salt-forming. In this way, the polypeptide molecule may exist in the salt form where it tended to be more stable during storage.

The salt-forming reaction referred to a reaction where an organic base or an organic acid formed a salt by respectively adding an alkali or an acid. Here, this kind of reaction specifically referred to the salt-forming reactions between the series of basic polypeptides (protamine-like peptides) of the present invention and the organic or inorganic acids. The salt included those generated by the reaction of the protamine-like peptides with the organic acid such as maleic acid, citric acid, tartaric acid, sulfonic acid, salicylic acid, malic acid and the like, or with the inorganic acid such as sulfuric acid, acetic acid, phosphoric acid, hydrochloric acid, carbonic acid and the like. The series of basic polypeptides (protamine-like peptides) underwent the salt-forming reaction to produce salts, mostly aiming at increasing the stability and solubility of drugs.

Methods:

1. Salt-forming reaction

The sulfate of protamine-like peptide was taken as an example here with the following specific test procedure.

A solution of 10 mg/ml of R15 in ethanol was prepared and a solution of 0.05% sulfuric acid in ethanol was added thereto. The mixture was shaken and centrifuged. The mixture was washed with ethanol twice and then centrifuged. Thereafter, the collected sediment was dissolved with water and lyophilized to yield an inorganic salt form of protamine-like peptide (R15 sulfate). By HPLC-UV, the resultant product was tested for quantity and purity. The results showed that the average yield of R15 hydrochloride prepared by this method was higher than 70% and the purity was higher than 98%. 2. The sulfate was taken as an example to illustrate the anti-heparin effect of the inorganic salt form of protamine-like peptide. Following the activity assay described above, the anti-heparin activities of R15 and its salt form at the same molar concentration were compared. The results showed that salt formation had no effect on the activity. The content of R15 sulfate was analyzed on its form before salt formation, so the weight of the sulfate radical was not taken into consideration. The results were shown in Table 8.

The test results showed that the sulfate form of R15 did not affect the anti-heparin effect. Also, other polypeptides in the inorganic acid salt and organic acid salt form were tested. As a result, the inorganic acid salt or the organic acid salt form of basic amino acid peptide (protamine-like peptide) had the same anti-heparin effect as the protamine-like peptides having the same number of amino acid residues.

Table 8. Comparison on Number of units of heparin neutralized by protamine-like peptides R15 and R15 sulfate in rabbit whole blood added with heparin and Anti-heparin index

| | Sequence | Amount of neutralized heparin (U/mg) | | | | | | Clotting time | I |
|---|---|---|---|---|---|---|---|---|---|
| | | 1 | 2 | 3 | 4 | 5 | Mean± SD | (min) | |
| Commercially available protamine | | 105 | 105 | 100 | 100 | 100 | 102±2.45 | 13.14±1.34 | 2.58 |
| R15 | $(R)_{15}$ | 115 | 110 | 110 | 110 | 115 | 112±2.74 | 12.69±0.44 | 6.27 |
| R15 sulfate | $(R)_{15}$ sulfate | 110 | 115 | 110 | 115 | 110 | 110±1.342 | 11.83±0.65 | 6.25 |
| Whole blood (blank control) | - | - | - | - | - | - | - | 10.62±0.49 | 3.94 |
| I = Anti-heparin index; R = Arginine; - = Not applicable or incalculable. | | | | | | | | | |

Test *6: In vivo* animal study on antagonism of protamine-like peptide R15 against excess heparin (for reference, not part of the claimed invention)

Materials:

A R15 sulfate was synthesized by the solid phase synthesis method. The purity was higher than 99.0% and the lot number was 20130321. The R15 sulfate had a good water solubility. After dissolved in water, the R15 sulfate was sub-packaged and lyophilized to yield white floccules.

Heparin sodium injection: Purchased from Tianjin Biochem Pharmaceutical Co., Ltd. with a State Medical Permitment No. of H12020505, the specification being 2 mL: 12.5 thousand units.

Protamine sulfate injection: Purchased from Yuekang Hyatt Beijing Pharmaceutical Group Co., Ltd., State Medical Permitment No. being H11020246, 09121002, expiration date being September 2015.

APTT kit (Lot No.: Y118081) and PT kit (Lot No.: Y103125) purchased from Shanghai Sunbio Medical Biotechnology Co., Ltd.; sodium citrate, Sigma.

Animals: 12 New Zealand rabbits, male (purchased from Military Academy of Medical Sciences Laboratory Animal Center). They were kept in our laboratory for a week and provided with water but no food overnight prior to the experiment.

Preparation of test solution of R15 sulfate

The test sample was in a powder form. A proper amount was accurately weighed on the basis of the dry sample and then dissolved in the normal saline to provide a solution containing 1 mg of test sample per mL.

Method:

The animals were divided into three groups with 4 animals per group. The groups were respectively referred to as the control group, the group involving commercially available protamine and the group involving R15 sulfate. The experimental procedure was shown in Table 9. The rabbits with big ears were weighed and then administrated with heparin injection (100 u/kg) through left auricular vein. Five minutes later, 1.35 ml of blood was collected through the right auricular vein and 0.15 ml of sodium citrate was added at a volume ratio of 9:1 for anticoagulation. The mixture was centrifuged at 3000 r/min for 15 min, and the plasma was taken for APTT and PT assays. Before 10 minutes relapsed, the protamine sulfate and R15 sulfate were respectively administrated at an amount of 1 mg/kg through the left auricular vein. As for the control group, the animals were injected an equal amount of normal saline. Five minutes later, 1.35 ml of blood was collected through the right auricular vein and 0.15 ml of sodium citrate was added at a volume ratio of 9:1 for anticoagulation. The mixture was centrifuged at 3000 r/min for 15 min, and the plasma was taken for APTT and PT assays.

Table 9. Test design and administration in each group

| Group | Number of animals | Heparinization | APTT and PT assays | Neutralization | APTT and PT assays |
|---|---|---|---|---|---|
| Control group | 4 | Normal saline | 1.35 ml of blood was collected through right auricular vein for APTT and PT assays | Normal saline | 1.35 ml of blood was collected through right auricular vein for APTT and PT assays |
| Group involving protamine sulfate (commercially available) | 4 | Heparin injection, 100 U/kg, *iv* | | Protamine sulfate, 1 mg/kg, *iv* | |
| Group involving R15 sulfate (Test drug) | 4 | | | R15 sulfate, 1 mg/kg, *iv* | |

Results and data processing:

Results were shown in Table 10. It can be seen from the results that the clotting time in the rabbit plasma added with heparin significantly differed from that in the control group with the clotting time being significantly prolonged. After the protamine-like peptide was used for neutralization, the clotting time returned to normal. Also, there was no significant difference between commercially available protamine and the sulfate of protamine-like peptide R15 functioning as the test drug (p>0.05). In addition, there was a significant difference between the group involving the sulfate of protamine-like peptide R15 and control group ($p<0.05$).

Table 10. Changes for clotting index in rabbit plasma added with heparin and Clotting index in rabbit plasma neutralized by protamine

| Test groups | Heparinization | | Neutralization by protamine | |
|---|---|---|---|---|
| | APTT | PT | APTT | PT |
| Control group | 32.63±2.09 | 12.25±1.78 | 32.63±2.09 | 12.25±1.78 |
| Group involving protamine sulfate (commercially available) | 160.25±3.71 | 64.26±2.83 | 42.06±1.43*; ** | 14.25±2.62*; ** |
| Group involving R15 sulfate (Test drug) | 158.23.25±3.35 | 66.51±2.58 | 44.69±1.98*,** | 15.11±2.69*,** |
| *Significant difference as compared to Group involving normal saline (p<0.05), ** No significant difference as compared to Group involving commercially available protamine (p>0.05). | | | | |

The test above showed that the protamine-like peptide of the present invention can be used as an alternative to protamine for eliminating and neutralizing heparin-induced bleeding tendency in clinical practice and had the potential to be developed in clinical application.

Test 7: Safety assessment on protamine-like peptide in an acute toxicity test

1 Materials and methods

1.1 Drug

Protamine-like peptides R15 (content: 96.54%).

1.2 Solvents

Normal saline produced by Shijiazhuang No.4 Pharmaceutical Co., Ltd.

1.3 Animals

Eighty-six Kunming mice (16 for pilot trial and 70 for formal test) with half being males were purchased from Military Academy of Medical Sciences Laboratory Animal Center and had a body weight of 20 to 25 g. They were kept under a 12L/12D photoperiod for one week before the formal test. Water was provided *ad libitum*, and the room was ventilated regularly with a humidity of 60%.

1.4 Method

1.4.1 Pilot trial

Sixteen healthy mice with a body weight of 18 to 22 g were selected which were not pregnant. They were provided with water but no food before the pilot trial. The protamine-like peptide was administrated at a dose (75 mg/kg) three times that of theoretically estimated one (25mg/kg). The test showed that the dose causing 100% mortality and the dose resulting in 0% mortality were respectively measured to be 50 mg / kg (all 3 test mice were dead) and 25 mg / kg (all 3 test mice survived). After administration, the mice were observed continuously for one week, and the general conditions such as the mental state as well as toxic response and death instances were recorded.

1.4.2 Formal test

1.4.2.1 Determination of dosage

Based on the doses causing 0% and 100% mortality, the animals were divided into 6 groups with doses being respectively 25, 30, 35, 40, 45, and 50 mg/kg by using a dose ratio of about 0.85,

1.4.2.2 Test method

Seventy healthy mice which were not pregnant were taken and provided with water but no food prior to the test. The mice were randomly divided into six groups (10 mice per group) based on the body weight and the gender with half mice in each group being males. One group was injected with normal saline, and the other five groups were administrated with protamine-like peptides with different concentrations. On the day of test, mice in test groups received one dose via injection into tail veins whereas mice in control group received the same volume of normal saline. After administration, the mice were observed consecutively for two weeks, and the general conditions such as the mental state as well as toxic response and death instances were recorded.

2 Results

2.1 Symptoms in acute intoxication

In all death cases, mice died within 2 hours after injection. In particular, most mice died within 30 min. In the groups with high doses (40mg / kg and 45mg / kg), mice died around 10 min after the injection. All the mice in the death cases experienced abnormal physiological responses within 2 to 5 minutes after injection, including lying still, reluctant to move; moving slowly, weak forelimbs, moving with hind legs only; unusual conditions such as violently driving legs, jumping, and raising the tail. Shortly after those abnormal physiological responses, mice died and the bodies were found rigid. Autopsy found that diaphragm turned to black and additionally most of heart was also found black, which were discovered in both male and female mice. The survived mice also experienced abnormal physiological responses within 2 to 5 minutes after injection, including lying still, reluctant to move, curling up in one place, and rarely struggling such as jumping and raising the tail. They generally returned to normal state after 12 hours, the normal state being not different from that in normal mice.

2.2 Median lethal dose

The Bliss method was used for the determination of median lethal dose (LD50), and the LD50 of protamine-like peptides administrated to mice via tail vein injection was measured to be 35.394 mg/kg with its 95% confidence limit (Feiller correction) being 32.177 to 38.473 mg/kg. The results were shown in Table 11.

Table 11. Median lethal dose calculated with Bliss method

| Dose Mg/kg | Logarithmic dose (x) | Number of animal (N) | Number of Death (N) | % Death (%) | Test probability unit (Y) | Regression probability unit (Y) |
|---|---|---|---|---|---|---|
| 50 | 1.699 | 10 | 10 | 1E2 | ---- | 6.6932 |
| 45 | 1.6532 | 10 | 8 | 80.00 | 5.8415 | 6.1768 |
| 40 | 1.6021 | 10 | 7 | 70.00 | 5.524 | 5.5996 |
| 35 | 1.5441 | 10 | 5 | 50.00 | 5 | 4.9452 |
| 30 | 1.4771 | 10 | 3 | 30.00 | 4.476 | 4.1897 |
| 25 | 1.3979 | 10 | 0 | 0.00 | ---- | 3.2962 |

Regression equation: y(Probit) = -12.479+11.285Log(D)
Median lethal dose LD50 = 35.394 mg/kg
LD50 (Feiller correction) 95% confidence limit =32.177 to 38.473 mg/kg
LD5 = 25.302 mg/kg
LD95 = 49.51 mg/kg

Discussion:

The synthetic protamine-like peptide had a LD50 of 35.394 mg/kg which was close to the median lethal dose of natural protamine (38 mg/kg) reported in literatures, indicating that protamine-like peptide had similar safety condition as the natural protamine.

The protamine-like peptides provided in the present invention have been described in detail through Examples and Tests. To sum up, it is known that:

1. The protamine-like peptides have well defined amino acid sequences and molecular formulae, and peptides with short chains have been selected with the optimal activity of heparin antagonism and/or neutralization. Thus, the antigenicity of this kind of exogenous peptide molecules can be reduced and the *in vivo* degradation and clearance rate of these peptides can be improved.

2. As the protamine-like peptide is a single kind of molecule, the dose-response relationship in terms of heparin antagonism and/or neutralization is stable and well defined and will not vary in different batches.

3. As the protamine-like peptide is a single kind of molecule, the product has a high purity and well defined quality standard.

4. The protamine-like peptide is used instead of protamine to effectively avoid clinical side effects and risks caused on human by exogenous macromolecular drugs.

Industrial applicability

The protamine-like peptide provided in the present invention (as set forth in the claims) is a polypeptide molecule which may be artificially designed and synthesized and thus are suitable for industrial production. This polypeptide molecule has the functions provided by the protamine. It neutralizes and antagonizes heparin in the blood and therefore could be used instead of protamine clinically to eliminate and neutralize heparin-induced bleeding tendency.

**Claims**

1. A protamine-like peptide or a pharmaceutical salt thereof for use in the treatment of eliminating and neutralizing bleeding tendency caused by heparin in blood, the protamine-like peptide being a linear polypeptide consisting of 9 to 20 Arginine (Arg, R) residues, wherein one Arginine residue at any one position is replaced with a Histidine (His, H) residue (R9-H to R20-H).

2. A protamine-like peptide or a pharmaceutical salt thereof for use in the treatment of eliminating and neutralizing bleeding tendency caused by heparin in blood, the protamine-like peptide being a linear polypeptide consisting of 9 to 20 Lysine (Lys, K) residues, wherein one Lysine residue at any one position is replaced with a Histidine residue (K9-H to K20-H).

3. A protamine-like peptide or a pharmaceutical salt thereof for use in the treatment of eliminating and neutralizing bleeding tendency caused by heparin in blood, the protamine-like peptide being a linear polypeptide consisting of a polypeptide with mixed types of amino acid residues, the polypeptide being formed by combining Lysine residue(s) (Lys, K) and Histidine residue(s) (His, H) with a linear polypeptide consisting of 10 to 20 Arginine residues (Arg, R), and the structure thereof being $(R)_5K_1H_1(R)_5$, $(R)_7K_1H_1(R)_7$, $(R)_9K_1H_1(R)_9$ or $(R)_{10}K_1H_1(R)_{10}$.

4. The protamine-like peptide or pharmaceutical salt thereof for use according to claim 1, wherein the protamine-like peptide consists of 14 Arginine residues and one Histidine residue (R15-H) and is derived from the protamine-like peptide consisting of 15 Arginine residues.

5. The protamine-like peptide or pharmaceutical salt thereof for use according to claim 4, wherein, the pharmaceutical salt of the protamine-like peptide comprises an inorganic salt such as sulfate, acetate, phosphate, hydrochlorate, carbonate or the like; or an organic salt such as maleate, citrate, tartrate, sulfonate, salicylate, malate or the like.

6. A protamine-like peptide which is one from the group consisting of R9-H, R15-H and R20-H, R9-H, R15-H and R20-H all consisting of Arginine residues and one Histidine residue.

7. The protamine-like peptide according to claim 6, wherein the structure of the R15-H is $(R)_1(H)_1(R)_{13}$, $(R)_4(H)_1(R)_{10}$, or $(R)7(H)_1(R)_7$.

8. A protamine-like peptide which is one from the group consisting of K9-H and K20-H, K9-H and K20-H both consisting of Lysine residues and one Histidine residue.

9. A protamine-like peptide which is a polypeptide with mixed types of amino acid residues, the polypeptide being formed by combining Lysine residue(s) (Lys, K) and Histidine residue(s) (His, H) with a linear polypeptide consisting of 10 to 20 Arginine residues (Arg, R), and the structure thereof being $(R)_5K_1H_1(R)_5$, $(R)_7K_1H_1(R)_7$, $(R)_9 K_1H_1(R)_9$ or $(R)_{10}K_1H_1(R)_{10}$.

10. A pharmaceutical salt derived from the protamine-like peptide according to any one of claims 6 to 9.

11. The pharmaceutical salt according to claim 10 comprising an inorganic salt such as sulfate, acetate, phosphate, hydrochlorate, carbonate or the like; or an organic salt such as maleate, citrate, tartrate, sulfonate, salicylate, malate or the like.

## Patentansprüche

1. Protamin-artiges Peptid (protamine-like peptide) oder pharmazeutisches Salz davon zur Verwendung bei der Behandlung der Beseitigung und Neutralisierung der Blutungstendenz, die durch Heparin im Blut hervorgerufen wird, wobei das Protamin-artige Peptid ein lineares Polypeptid bestehend aus 9 bis 20 Argininresten (Arg, R) ist, wobei ein Argininrest an einer beliebigen Position mit einem Histidinrest (His, H) ersetzt ist (R9-H bis R20-H).

2. Protamin-artiges Peptid oder pharmazeutisches Salz davon zur Verwendung bei der Behandlung der Beseitigung und Neutralisierung der Blutungstendenz, die durch Heparin im Blut hervorgerufen wird, wobei das Protamin-artige Peptid ein lineares Polypeptid bestehend aus 9 bis 20 Lysinresten (Lys, K) ist, wobei ein Lysinrest an einer beliebigen Position mit einem Histidinrest ersetzt ist (K9-H bis K20-H).

3. Protamin-artiges Peptid oder pharmazeutisches Salz davon zur Verwendung bei der Behandlung der Beseitigung und Neutralisierung der Blutungstendenz, die durch Heparin im Blut hervorgerufen wird, wobei das Protamin-artige Peptid ein lineares Polypeptid bestehend aus einem Polypeptid mit gemischten Arten von Aminosäureresten ist, wobei das Polypeptid durch Kombinieren von (einem) Lysinrest (en) (Lys, K) und (einem) Histidinrest (en) (His, H) mit einem linearen Polypeptid bestehend aus 10 bis 20 Argininresten (Arg, R) gebildet ist, und die Struktur davon $(R)_5K_1H_1(R)_5$, $(R)_7K_1H_1(R)_7$, $(R)_9K_1H_1(R)_9$ oder $(R)_{10}K_1H_1(R)_{10}$ ist.

4. Protamin-artiges Peptid oder pharmazeutisches Salz davon zur Verwendung nach Anspruch 1, wobei das Protamin-artige Peptid aus 14 Argininresten und einem Histidinrest (R15-H) besteht und von einem Protamin-artigen Peptid bestehend aus 15 Argininresten stammt.

5. Protamin-artiges Peptid oder pharmazeutisches Salz davon zur Verwendung nach Anspruch 4, wobei das pharmazeutische Salz des Protamin-artigen Peptids ein anorganisches Salz wie Sulfat, Acetat, Phosphat, Hydrochlorat, Carbonat oder Ähnliches; oder ein organisches Salz wie Maleat, Citrat, Tartrat, Sulfonat, Salicylat, Malat oder Ähnliches umfasst.

6. Protamin-artiges Peptid, das eines aus der Gruppe bestehend aus R9-H, R15-H und R20-H ist, wobei R9-H, R15-H und R20-H alle aus Argininresten und einem Histidinrest bestehen.

7. Protamin-artiges Peptid nach Anspruch 6, wobei die Struktur des R15-H $(R)_1(H)_1(R)_{13}$, $(R)_4(H)_1(R)_{10}$ oder $(R)_7(H)_1(R)_7$ ist.

8. Protamin-artiges Peptid, das eines aus der Gruppe bestehend aus K9-H und K20-H ist, wobei K9-H und K20-H beide aus Lysinresten und einem Histidinrest bestehen.

9. Protamin-artiges Peptid, das ein Polypeptid mit gemischten Arten an Aminosäureresten ist, wobei das Polypeptid durch Kombinieren von (einem) Lysinrest (en) (Lys, K) und (einem) Histidinrest (en) (His, H) mit einem linearen Polypeptid bestehend aus 10 bis 20 Argininresten (Arg, R) gebildet ist, und die Struktur davon $(R)_5K_1H_1(R)_5$, $(R)_7K_1H_1(R)_7$, $(R)_9K_1H_1(R)_9$ oder $(R)_{10}K_1H_1(R)_{10}$ ist.

10. Pharmazeutisches Salz, das von dem Protamin-artigen Peptid nach einem der Ansprüche 6 bis 9 stammt.

11. Pharmazeutisches Salz nach Anspruch 10, umfassend ein anorganisches Salz wie Sulfat, Acetat, Phosphat, Hydrochlorat, Carbonat oder Ähnliches; oder ein organisches Salz wie Maleat, Citrat, Tartrat, Sulfonat, Salicylat, Malat oder Ähnliches.

**Revendications**

1. Peptidomimétique de la protamine ou un sel pharmaceutique de celui-ci pour une utilisation dans le traitement d'élimination et de neutralisation de la tendance à l'hémorragie causée par l'héparine dans le sang, le peptidomimétique de la protamine étant un polypeptide linéaire constitué de 9 à 20 résidus Arginine (Arg, R), dans lequel un résidu Arginine au niveau d'une position quelconque est remplacé par un résidu Histidine (His, H) (R9-H à R20-H).

2. Peptidomimétique de la protamine ou un sel pharmaceutique de celui-ci pour une utilisation dans le traitement d'élimination et de neutralisation de la tendance à l'hémorragie causée par l'héparine dans le sang, le peptidomimétique de la protamine étant un polypeptide linéaire constitué de 9 à 20 résidus Lysine (Lys, K), dans lequel un résidu Lysine au niveau d'une position quelconque est remplacé par un résidu Histidine (K9-H à K20-H).

3. Peptidomimétique de la protamine ou un sel pharmaceutique de celui-ci pour une utilisation dans le traitement d'élimination et de neutralisation de la tendance à l'hémorragie causée par l'héparine dans le sang, le peptidomimétique de la protamine étant un polypeptide linéaire constitué d'un polypeptide avec des types mixtes de résidus d'acides aminés, le polypeptide étant formé par combinaison de résidu (s) Lysine (Lys, K) et de résidu (s) Histidine (His, H) avec un polypeptide linéaire constitué de 10 à 20 résidus Arginine (Arg, R), et la structure de celui-ci étant $(R)_5 K_1 H_1 (R)_5$, $(R)_7 K_1 H_1 (R)_7$, $(R)_9 K_1 H_1 (R)_9$ ou $(R)_{10} K_1 H_1 (R)_{10}$.

4. Peptidomimétique de la protamine ou sel pharmaceutique de celui-ci pour une utilisation selon la revendication 1, dans lequel le peptidomimétique de la protamine est constitué de 14 résidus Arginine et d'un résidu Histidine (R15-H) et est dérivé du peptidomimétique de la protamine constitué de 15 résidus Arginine.

5. Peptidomimétique de la protamine ou sel pharmaceutique de celui-ci pour une utilisation selon la revendication 4, dans lequel le sel pharmaceutique du peptidomimétique de la protamine comprend un sel inorganique tel qu'un sulfate, un acétate, un phosphate, un chlorhydrate, un carbonate ou autre ; ou un sel organique tel qu'un maléate, un citrate, un tartrate, un sulfonate, un salicylate, un malate ou autre.

6. Peptidomimétique de la protamine qui est l'un du groupe constitué de R9-H, R15-H et R20-H, R9-H, R15-H et R20-H tous constitués de résidus Arginine et d'un résidu Histidine.

7. Peptidomimétique de la protamine selon la revendication 6, dans lequel la structure du R15-H est $(R)_1 (H)_1 (R)_{13}$, $(R)_4 (H)_1 (R)_{10}$ ou $(R)_7 (H)_1 (R)_7$.

8. Peptidomimétique de la protamine qui est l'un du groupe constitué de K9-H et K20-H, K9-H et K20-H tous deux constitués de résidus Lysine et d'un résidu Histidine.

9. Peptidomimétique de la protamine qui est un polypeptide avec des types mixtes de résidus d'acides aminés, le polypeptide étant formé par combinaison de résidu (s) Lysine (Lys, K) et de résidu (s) Histidine (His, H) avec un polypeptide linéaire constitué de 10 à 20 résidus Arginine (Arg, R), et la structure de celui-ci étant $(R)_5 K_1 H_1 (R)_5$, $(R)_7 K_1 H_1 (R)_7$, $(R)_9 K_1 H_1 (R)_9$ ou $(R)_{10} K_1 H_1 (R)_{10}$.

10. Sel pharmaceutique dérivé du peptidomimétique de la protamine selon l'une quelconque des revendications 6 à 9.

11. Sel pharmaceutique selon la revendication 10 comprenant un sel inorganique tel qu'un sulfate, un acétate, un phosphate, un chlorhydrate, un carbonate ou autre ; ou un sel organique tel qu'un maléate, un citrate, un tartrate, un sulfonate, un salicylate, un malate ou autre.

$$C_{90}H_{182}N_{60}O_{16}$$
Exact Mass: 2359.53
Mol. Wt.: 2360.8

Figure 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Research and Development of Polypeptide Drugs. People's Medical Publishing House, Co., Ltd, July 2011
- Bioassay of Protamine Sulfate from China Standard Operation Procedure for Drug Test. Pharmaceutical Press, 2010

- **R.B. MERRIFIELD.** *American scientist,* 1963
- **CHILLEMI F. ; MERRIFIELD R B.** Use of N-im-dinitrophenylhistidine in the solid-phase synthesis of the tricosapeptides124-146 of human hemoglobin. *Biochemistry,* 1969, vol. 8, 4344